# EUROPÄISCHE PATENTSCHRIFT

(11) **EP 2 798 348 B1**
(45) Veröffentlichungstag und Bekanntmachung des Hinweises auf die Patenterteilung: **02.08.2017**
(21) Anmeldenummer: 13702375.0
(22) Anmeldetag: 21.01.2013
(51) Int. Cl.: G01N 33/487, G01N 27/327

(54) **ANORDNUNG UND VERFAHREN ZUR ELEKTROCHEMISCHEN ANALYSE VON FLÜSSIGEN PROBEN MIT LATERAL FLOW ASSAYS**
ARRANGEMENT AND METHOD FOR THE ELECTROCHEMICAL ANALYSIS OF LIQUID SAMPLES BY MEANS OF LATERAL FLOW ASSAYS
DISPOSITIF ET PROCÉDÉ D'ANALYSE ÉLECTROCHIMIQUE D'ÉCHANTILLONS LIQUIDES À L'AIDE DE SYSTÈMES DE TEST À FLUX LATÉRAL

(30) Priorität: 08.02.2012 DE 102012201843
(43) Veröffentlichungstag der Anmeldung: 05.11.2014
(73) Patentinhaber: Siemens Aktiengesellschaft, 80333 München (DE)
(72) Erfinder: GUMBRECHT, Walter, 91074 Herzogenaurach (DE); PAULICKA, Peter, 91341 Röttenbach (DE)
(86) Internationale Anmeldenummer: PCT/EP2013/051026
(87) Internationale Veröffentlichungsnummer: WO 2013/117413

(56) Entgegenhaltungen:
- EP-A2- 0 271 102
- WO-A1-2010/102279
- DE-A1- 19 631 530

## Beschreibung

Die vorliegende Erfindung bezieht sich auf eine Anordnung und ein Verfahren zur elektrischen Detektion von flüssigen Proben mit Lateral Flow Assays gemäß den Merkmalen gemäß dem Oberbegriff des Patentanspruchs 1. Eine derartige Anordnung und ein derartiges Verfahren sind aus der Offenlegungsschrift WO 2010/102279 A1 bekannt.

Lateral Flow Assays sind in der In-vitro-Diagnostik (IVD) weit verbreitet. Sie sind einfach in der Handhabung und sehr kostengünstig. Nachteilig an Lateral Flow Assays ist eine geringe Sensitivität, eine geringe Multiplexität und eine schlechte Quantifizierbarkeit der Ergebnisse.

Eine gute Quantifizierbarkeit ist mit optischen, magnetischen und elektrischen Verfahren zu erreichen, bisher jedoch bei sehr niedriger Multiplexität, d.h. gleichzeitiger bzw. simultaner Messung an mehreren räumlich getrennten Messpunkten.

Aus der US 6,896,778 ist eine Anordnung bekannt, bei welcher für eine gute Multiplexität Goldelektroden über Ausnehmungen eines elektrisch isolierenden Trägers als Array angeordnet sind, und die Ausnehmungen mit voneinander räumlich getrennten Membranen aus einem Polymer/Mikrofiber-Matrix Material befüllt sind. Die Membrane sind ionenselektiv, und z.B. für Immuno-Sensoren in Immuno-Assays nicht geeignet.

Bei Verwendung von Fänger-Antikörpern in Immuno-Assays müssen diese direkt auf den Goldelektroden bzw. -Sensoren immobilisiert werden. Bei einer Anordnung, analog der in US 6,896,778 beschriebenen laminierten Anordnung eines Trägers aus Isolatorschichten und Goldelektroden, wobei die Goldelektroden über Ausnehmungen in den Isolatorschichten in Array-Form angeordnet sind, ergibt sich jeweils eine kleine Kavität über den Goldelektroden durch die umgebende Isolatorschicht. Bei Beaufschlagung mit Flüssigkeit direkt oder über ein Lateral Flow Papier als Membrane über der Anordnung entstehen Luftblasen im Bereich der Kavitäten, welche bei einer Messung zu einem Ausfall der jeweiligen Elektroden mit Lufteinschlüssen führen.

Darüber hinaus sind aus der deutschen Offenlegungsschrift DE 196 31 530 A1 und der europäischen Offenlegungsschrift EP 0 271 102 A2 Sensoren zu Messung von Ionenkonzentrationen bekannt.

Aufgabe der vorliegenden Erfindung ist es deshalb, eine Anordnung und ein Verfahren zur elektrischen Detektion von flüssigen Proben anzugeben, welche eine gute Multiplexität bei sehr guter Sensitivität und Quantifizierbarkeit ermöglichen. Unter guter Multiplexität ist dabei eine Multiplexität z.B. im Bereich von 3 bis 10-plex (Sensoren) zu verstehen. Insbesondere ist es Aufgabe eine Anordnung und ein Verfahren anzugeben, welche eine zuverlässige Messung ermöglichen, insbesondere ohne störende Lufteinschlüsse über den Elektroden.

Die angegebene Aufgabe wird bezüglich der Anordnung zur elektrischen Detektion von flüssigen Proben mit Lateral Flow Assays mit den Merkmalen des Anspruchs 1 und bezüglich des Verfahrens zur elektrischen Detektion von flüssigen Proben mit der zuvor beschriebenen Anordnung mit den Merkmalen des Anspruchs 9 gelöst.

Vorteilhafte Ausgestaltungen der erfindungsgemäßen Anordnung zur elektrischen Detektion von flüssigen Proben mit Lateral Flow Assays und des Verfahrens zur elektrischen Detektion von flüssigen Proben mit der zuvor beschriebenen Anordnung gehen aus den jeweils zugeordneten abhängigen Unteransprüchen hervor. Dabei können die Merkmale des Hauptanspruchs mit Merkmalen der Unteransprüche und Merkmale der Unteransprüche untereinander kombiniert werden.

Die erfindungsgemäße Anordnung zur elektrischen Detektion von flüssigen Proben mit Lateral Flow Assays umfasst eine Membrane, welche auf einer Vorderseite eines ersten Trägers angeordnet ist. Der erste Träger ist elektrisch isolierend ausgebildet und auf dem ersten Träger sind elektrisch leitende Elektroden ausgebildet. Die Elektroden auf der Vorderseite des ersten Trägers sind zwischen dem Träger und der Membrane, in direktem Kontakt zur Membrane angeordnet. Der Träger weist jeweils im Bereich einer jeweiligen Elektrode eine durch seine Dicke, von der Vorderseite zur Rückseite durchgehende Öffnung auf, durch welche die Elektrode leitend von der Vorderseite zur Rückseite des Trägers elektrisch kontaktiert ist; dadurch lassen sich elektrische Kurzschlüsse zwischen Elektrodenkontakten z.B. bei Kontakt mit Probenflüssigkeit auf der Vorderseite des Trägers verhindern.

Durch die Anordnung der Elektroden auf der Vorderseite des ersten Trägers, d.h. auf der Seite, auf welcher auch die Membrane angeordnet ist, können die Elektroden einen direkten Kontakt zu der Membrane ausbilden. Die Ausbildung eines Hohlraums bzw. einer Kavität, wie sie z.B. im zuvor beschriebenen Stand der Technik vorhanden ist, wird dadurch verhindert. Die Kontaktfläche wird bei flach auf der Elektrode angeordneter Membrane maximiert und bei Beaufschlagung der flüssigen, zu analysierenden Probe auf die Membrane stehen die Elektroden in direktem Kontakt zur flüssigen Probe. Luftblasen bzw. Lufteinschlüsse, welche eine Messung behindern oder vollständig verhindern können, werden durch den direkten Kontakt von Elektroden und Membrane, und somit auch dem direkten Kontakt von Elektroden und der flüssigen Probe, verhindert. Dadurch wird eine zuverlässige Messung der Probe ermöglicht, auch bei Multiplex-Messung (mit mehreren Sensoren gleichzeitig), mit sehr guter Sensitivität und Quantifizierbarkeit der Probe.

Die Membrane kann als eine geschlossene Schicht ausbildet sein, über welche die Elektroden, insbesondere alle Elektroden miteinander verbunden sind. Dadurch ist ein lateraler Flüssigkeitstransport (Lateral Flow) vollständig über die Membrane insbesondere über alle Elektroden möglich.

Die Membrane kann ein Lateral Flow Papier, insbesondere aus Nitrocellulose umfassen oder sein. Lateral Flow Papier weist eine hohe Porosität auf und saugt die flüssige Probe gut auf sowie transportiert diese gut zu den Elektroden und führt dort zu einer guten Benetzung der Elektroden mit der zu untersuchenden Probenflüssigkeit. Ein guter elektrischer Kontakt über mit flüssiger Probe vollgesaugter Membrane zwischen Elektroden wird so ermöglicht. Nitrocellulose ist kostengünstig und weist als Membrane verwendet die zuvor beschriebenen Eigenschaften auf.

Die Elektroden können Metallelektroden sein, insbesondere aus Gold. Elektroden aus Metall sind in der Regel relativ stabil, und insbesondere Goldelektroden sind elektrochemisch gut zu verwenden, da sie zu zeitlich stabilen Messsignalen führen können und chemisch im Wesentlichen innert sind.

Der Träger kann mehrere isolierende Schichten umfassen, insbesondere Schichten aus Polymeren und/oder Schichten, welche miteinander durch laminieren verbunden sind. Laminierte Träger aus Polymeren sind z.B. Leiterplatten, welche kostengünstig herzustellen sind.

Die Membrane können sandwichartig angeordnet sein, einmal zwischen der Vorderseite des ersten Trägers in direktem Kontakt mit den elektrisch leitenden Elektroden (Arbeitselektroden) des ersten Trägers, und zweitens einer Rückseite eines zweiten Trägers in direktem Kontakt mit wenigstens einer Elektrode (Gegenelektrode), insbesondere genau einer Elektrode auf der Rückseite des zweiten Trägers. Diese Anordnung ermöglicht einen kompakten Aufbau und kurze Wege über die Membrane zwischen Elektroden, um eine Spannung zwischen den Arbeits- und der Gegenelektrode aufzubauen.

Jede Elektrode auf der Vorderseite des ersten Trägers kann jeweils mit der wenigstens einen Elektrode auf der Rückseite des zweiten Trägers elektrisch verbunden sein, insbesondere über ein elektrisches Messinstrument bzw. Messeinrichtung zur Messung von Strom und/oder Spannung und/oder Kapazität. Die Elektroden können auf der Vorderseite des ersten Trägers in einer Reihe hintereinander oder in Array-Form angeordnet sein. Dadurch wird eine elektrochemische Messung ermöglicht und eine räumlich aufgelöste elektrochemische Messung der flüssigen Probe in der Membrane analog einer optischen Messung in der Chromatographie durchführbar.

Das erfindungsgemäße Verfahren zur elektrischen Detektion von flüssigen Proben erfolgt mit einer zuvor beschriebenen Anordnung. Die flüssige Probe wird auf die Membran aufgebracht und über insbesondere Kapillarkräfte über die Membran zu den Elektroden bewegt. Die Membrane verbindet die Elektroden untereinander, insbesondere elektrochemisch wenn die Membrane mit Flüssigkeit befüllt ist. Insbesondere genau eine Membrane verbindet insbesondere alle Elektroden untereinander. Dadurch ist eine gute Leitfähigkeit bei leitfähiger Flüssigkeit zwischen den Elektroden über die eine Membrane gewährleistet.

Die Anordnung bewirkt nach dem Lateral Flow Verfahren eine räumliche und/oder zeitliche Auftrennung von Substanzen in der flüssigen Probe analog der Chromatographie oder mit unterschiedlichen Fängermolekülen an unterschiedlichen Orten immobilisiert. Die räumliche und/oder zeitliche Auftrennung kann mit den Elektroden in Form von Strom- und/oder Spannungs- und/oder Ladungsänderungen elektrochemisch gemessen werden.

Bei dem beschriebenen Aufbau kann die Membrane in direktem Kontakt mit den Elektroden stehen. Jeweils die Kontaktfläche einer jeden Elektrode mit der Membrane kann so vollständig mit der flüssigen Probe benetzt werden, insbesondere ohne Lufteinschlüsse über der Elektrode. Dadurch wird eine gute elektrochemische Messung mit der Elektrode gewährleistet, welche z.B. durch Luftblasen direkt über der Elektrode verhindert oder zumindest behindert würde.

Die flüssige Probe kann eine biochemische Probe, insbesondere eine Körperflüssigkeit sein. So können z.B. Urin, Blut, oder deren Aufschlüsse untersucht werden.

Die mit dem Verfahren zur elektrischen Detektion von flüssigen Proben mit der zuvor beschriebenen Anordnung verbundenen Vorteile sind analog den Vorteilen, welche zuvor im Bezug auf die Anordnung zur elektrischen Detektion von flüssigen Proben mit Lateral Flow Assays beschrieben wurden.

Bevorzugte Ausführungsformen der Erfindung mit vorteilhaften Weiterbildungen gemäß den Merkmalen der abhängigen Ansprüche werden nachfolgend anhand der Figuren näher erläutert, ohne jedoch darauf beschränkt zu sein.

Es wird in den Figuren dargestellt:
- Fig. 1: eine schematische Schnittdarstellung durch eine Anordnung 1 zur elektrischen Detektion von flüssigen Proben 5 mit einer laminierten Elektrode 3 und einer darüber angeordneten ionenselektiven Membrane 4 nach dem Stand der Technik, und
- Fig. 2: eine schematische Schnittdarstellung durch eine erfindungsgemäße Anordnung 1 zur elektrischen Detektion von flüssigen Proben 5 mit einer laminierten Elektrode 3 für Lateral Flow Assays, wobei die Elektrode 3 auf der Vorderseite eines Träger 2 zwischen Träger 2 und Membrane 4 angeordnet ist, und
- Fig. 3: eine schematische Schnittdarstellung durch eine Anordnung 1 analog der in Fig. 2, mit mehreren Elektroden 3 in Reihe hintereinander angeordnet und ohne elektrischen Kontakt 6 von der Rückseite der Anordnung 1 her, und
- Fig. 4.: eine schematische Darstellung in Aufsicht auf die in Fig. 3 gezeigte Anordnung 1 mit einem Streifen Lateral Flow Papier 4 derart aufgelegt auf die Elektroden 3, dass ein seitlicher Bereich für einen elektrischen Kontakt 6 frei bleibt, und
- Fig. 5: eine schematische Schnittdarstellung durch eine Anordnung 1 analog der in Fig. 3 gezeigten, mit einem zweiten Elektroden-Träger 8 mit Gegenelektrode 9 auf der Membrane 4 angeordnet und Messeinrichtungen 10 jeweils verbunden mit einer Arbeitselektrode 3 des ersten Trägers 2 und der Gegenelektrode 9 des zweiten Trägers 8.

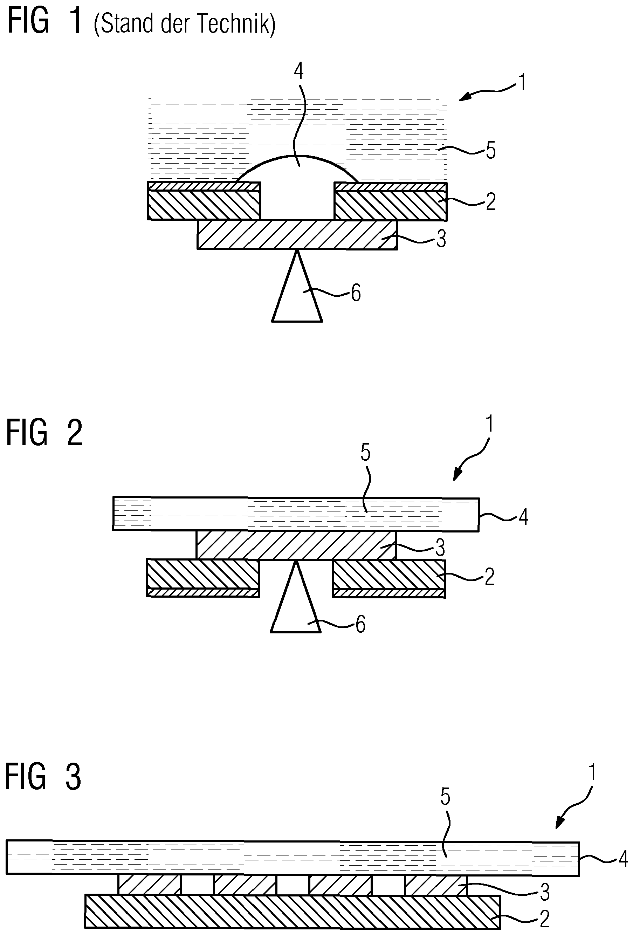

Die in der Fig. 1 gezeigte Anordnung 1 zur elektrischen Detektion von flüssigen Proben 5 nach dem Stand der Technik weist eine laminierte Elektrode 3 eines Elektroden-Arrays auf. Die Anordnung 1 ist schematisch in Schnittdarstellung gezeigt. Die Elektrode 3 ist unter einem ersten Träger 2 angeordnet, welcher z.B. aus laminierten Polymerschichten analog einer Leiterplatte aufgebaut ist, wobei die Elektrode 3 z.B. als Goldschicht auf den Träger 2 auflaminiert ist. Die Elektrode kann aber auch z.B. aufgeklebt sein oder galvanisch aufgebracht sein.

Im Träger 2, oberhalb der Elektrode 3 ist eine durch den Träger 2 durchgehende Ausnehmung eingebracht. Diese kann z.B. in Form einer Bohrung oder Fräsung z.B. kreisrund ausgebildet sein. In der Ausnehmung, in Kontakt mit der Elektrode 3 ist eine Membrane 4 als Ionenselektive Schicht angeordnet, welche die freie Fläche der Elektrode 3 in der Ausnehmung vollständig bedeckt.

Die Elektrode 3 ist elektrisch über einen elektrischen Kontakt 6 von der Rückseite her kontaktiert, d.h. von der Seite der Elektrode 3 her, welche der Membrane 4 gegenüber liegt und nicht von der Membrane 4 bedeckt ist. Über die Membrane 4 und die Seite des Trägers 2, auf welcher keine Elektroden 3 angeordnet sind und die der Seite mit den Elektroden auf dem Träger 2 gegenüber liegt, wird eine flüssige Probe 5 geleitet, welche über die Membrane 4 mit der Elektrode 3 elektrochemisch in Kontakt steht. Das bedeutet, Ionen können durch die Membrane 4 aus der flüssigen Probe 5 zur Elektrode 3 sich bewegen.

Eine der Einfachheit halber nicht gezeigte Gegenelektrode ist mit der flüssigen Probe 5 in elektrischem Kontakt. Zwischen der Gegenelektrode und der Elektrode 3, welche als Arbeitselektrode fungiert, kann über eine nicht gezeigte Messeinrichtung 10 ein Strom-, eine Spannungs- und/oder eine Ladungsänderung an der Elektrode 3 gemessen werden. Die Messung erfolgt abhängig der flüssigen Probe 5 und dient der Analyse der Probe 5. Die Messung kann abhängig vom Probenfluss und/oder der Zeit erfolgen und gibt Aufschluss über die Zusammensetzung bzw. chemische/biochemische Bestandteile der Probe 5. Als Proben 5 können z.B. Blut, Urin oder andere Körperflüssigkeiten dienen. Es können aber auch andere Flüssigkeiten bis hin zu Gasen untersucht werden.

Die in Fig. 1 gezeigte Anordnung 1 kann in einem Sensor-Array oder einer anderen Anordnung von Sensoren, z.B. in Reihe verwendet werden. Die Elektroden 3 stellen die Sensoren dar. Für eine zuverlässige Messung ist wichtig, dass keine Flüssigkeit 5 auf die Seite des ersten Trägers 2 mit den Elektroden 3 gelangt, auf welcher die elektrischen Kontakte 6 liegen.

Ein Nachteil des zuvor beschriebenen Aufbaus ist die ionenselektive Membrane 4. Die Membrane 4 ist z.B. für Immuno-Sensoren nicht geeignet. Der komplizierte Aufbau ist aufwendig herzustellen und schwer zu handhaben.

In Fig. 2 ist eine schematische Schnittdarstellung durch eine erfindungsgemäße Anordnung 1 zur elektrischen Detektion von flüssigen Proben 5 dargestellt. Auf einem ersten, laminierten Träger 2 ist eine Elektrode 3 angeordnet, analog der zuvor unter dem Stand der Technik beschriebenen Anordnung 1. Die Membrane 4 ist auf der Vorderseite der Elektrode 3 angeordnet, welche erfindungsgemäß der Seite der Elektrode 3 in Kontakt mit dem Träger 2 gegenüber liegt. Die Membrane 4 ist z.B. Lateral Flow Papier, welches flach auf der Elektrode aufliegt.

In dem Ausführungsbeispiel, welches in Fig. 2 dargestellt ist, wird die Elektrode 3 elektrisch über einen elektrischen Kontakt 6 auf der Rückseite der Elektrode 3 kontaktiert. Im laminierten Träger 2 ist im Bereich der Elektrode 3 auf deren Rückseite eine Ausnehmung, durch welche hindurch der elektrische Kontakt 6 bis zur Elektrode 3 ragt. Wie im Weiteren beschrieben wird, kann die Elektrode 3 auch von der Vorderseite her kontaktiert werden.

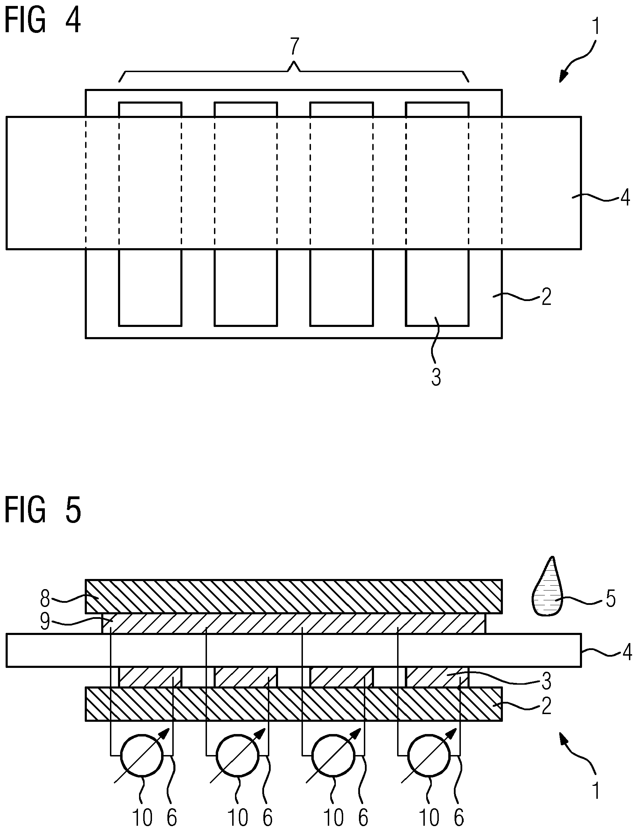

Fig. 3 und Fig. 4 zeigen ein Ausführungsbeispiel der erfindungsgemäßen Anordnung 1, bei welchem ein elektrischer Kontakt 6 der Elektroden 3 seitlich auf der Vorderseite erfolgen kann. In Fig. 3 ist eine schematische Schnittdarstellung durch eine Anordnung 1 analog der in Fig. 2 gezeigten dargestellt, aber mit mehreren der in Fig. 2 gezeigten Elektroden 3 in Reihe hintereinander angeordnet. Die Elektroden 3 können auch als Array, z.B. matrixförmig auf dem ersten Träger 2 angeordnet sein.

In Fig. 4 ist eine Aufsicht auf die in Fig. 3 gezeigte Anordnung 1 mit Elektroden 3 in einer Reihe 7 dargestellt. Eine Membrane 4, z.B. aus Lateral Flow Papier ist in Streifenform derart auf den Elektroden 3 angeordnet, dass ein seitlicher Bereich der Elektroden 3 für einen elektrischen Kontakt frei bleibt. In dem seitlichen Bereich können jeweils an jeder Elektrode 3 elektrische Kontakte 6 angebracht werden, was der Einfachheit halber in den Figuren nicht dargestellt ist.

In Fig. 5 ist ein Aufbau für eine elektrische Messung mit der in den Fig. 3 und 4 gezeigten erfindungsgemäßen Anordnung 1 in schematischer Schnittdarstellung gezeigt. Auf der Membrane 4 ist ein zweiter Träger 8 mit einer Elektrode 9 angeordnet, wobei die Membrane 4 in direktem Kontakt flach an der Elektrode 9 anliegt. Die Elektrode 9 dient als Gegenelektrode. Unter der Membrane 4 ist eine Reihe von Elektroden 3, welche als Arbeitselektroden dienen, in direktem Kontakt mit der Membrane 4 angeordnet. Die Elektroden 3 sind, wie schon in den Fig. 2 bis 4 gezeigt, auf einem Träger 2 angeordnet. Somit liegt die Membrane 4 sandwichartig zwischen den Elektroden 3 auf dem ersten Träger 2 und der Elektrode 9, welche flächig auf dem zweiten Träger 8 aufgebracht ist.

Wie in Fig. 5 schematisch gezeigt, kann für eine Messung die Elektrode 9 jeweils über ein elektrisches Messgerät bzw. eine Messeinrichtung 10 mit jeder Elektrode 3 elektrisch verbunden sein. Dabei können die Elektroden 3, 9 wie in Fig. 2 gezeigt von der Seite mit dem Träger 2, 8 her durch Öffnungen im Träger 2, 8 elektrisch kontaktiert werden, oder wie für Fig. 4 beschrieben von der Seite der Elektroden 3, 9 her, welche in direktem Kontakt mit der Membrane 4 steht, jedoch in einem Bereich der Elektroden 3, 7, in dem die Membrane 4 nicht angeordnet ist.

Wird die Membrane 4, z.B. Lateral Flow Papier aus Nitrocellulose in Streifenform oder in einer anderen Form, mit einer flüssigen Probe 5, z.B. Blut oder Urin beaufschlagt, so wird z.B. durch die poröse Struktur der Membrane 4 die Probe 5 in und durch die Membrane 4 bewegt. Die Membrane 4 wird somit mit der Probe 5 "befüllt". Durch die elektrische Leitfähigkeit der Probe 5 sind die mit der Membrane 4 in direktem Kontakt stehenden Elektroden 3, 9 elektrisch bzw. elektrochemisch miteinander verbunden, und jeweils über die Messeinrichtung 10 zwischen der jeweiligen Elektrode 3 als Arbeitselektrode und der Elektrode 9 als Gegenelektrode ist ein geschlossener Stromkreis gegeben. Mit elektrochemischen Messungen kann bei einem Elektroden-Array oder in Reihe angeordneten Elektroden 3, räumlich und zeitlich aufgelöst die Zusammensetzung der flüssigen Probe untersucht werden. So können an den einzelnen Elektroden 3 ortsbezogen, d.h. am Ort der Elektrode 3, elektrochemische Messungen über Strom-, Spannungs- und/oder Kapazitätsmessungen Aufschluss über die an dem Ort befindliche Probe 5 geben.

Analog chromatographischen Untersuchungen kann die Probenzusammensetzung analysiert werden oder Fänger-Moleküle können auf der jeweiligen Elektrode 3 immobilisiert werden, z.B. auf unterschiedlichen Elektroden 3 unterschiedliche Fängermoleküle, und den Nachweis einzelner Substanzen in der Probe 5 ermöglichen. Die Fänger-Moleküle können auch im Bereich über der Elektrode 3 in der Membran 4 räumlich verteilt immobilisiert sein. Dadurch ist die erfindungsgemäße Anordnung 1 in Immuno-Assays zu verwenden.

Die Elektroden 3, 9 können wie in den Figuren dargestellt, siehe insbesondere Fig. 5, in einem Messaufbau mit Arbeits- 3 und Gegenelektrode 9 verwendet werden. Zusätzlich kann auch wenigstens eine, der Einfachheit halber in den Figuren nicht dargestellte Referenzelektrode RE verwendet werden. Als Elektroden können Metallschichten, insbesondere aus Gold oder Platin, verwendet werden oder z.B. als Referenzelektrode Silber/Silber-Chlorid-Schichten oder -Elektroden. Auch andere in der Elektrochemie übliche Messaufbauten sind möglich.

Die zuvor beschriebenen Ausführungsbeispiele können in Kombination verwendet werden. Die Ausführungsbeispiele können auch mit Ausführungsbeispielen, bekannt aus dem Stand der Technik kombiniert werden. So können als Membrane 4 neben Lateral Flow Papier z.B. aus Nitrocellulose auch Membrane 4 aus Polyvinylidene Fluriden, elektrostatisch behandeltem Nylon oder Polyethersulfone verwendet werden. Elektroden 3, 9 können flächig auf dem Träger 2, 8 aufgebracht sein oder räumlich strukturiert, in Reihe hintereinander, in Array-Form in n x m -Matrix aus n Zeilen und m Spalten, oder auch mit unterschiedlichen Höhenprofilen versehen sein.

## Patentansprüche

1. Anordnung (1) zur elektrischen Detektion von flüssigen Proben (5) mit Lateral Flow Assays, wobei das Lateral Flow Assay eine Membrane (4) umfasst, welche auf einer Vorderseite eines ersten Trägers (2) angeordnet ist, und wobei der erste Träger (2) elektrisch isolierend ausgebildet ist und auf dem ersten Träger (2) elektrisch leitende Elektroden (3) angeordnet sind, und wobei die Elektroden (3) auf der Vorderseite des ersten Trägers (2) zwischen dem Träger (2) und der Membrane (4), in direktem Kontakt zur Membrane (4) angeordnet sind,
**dadurch gekennzeichnet, dass**
der Träger (2, 8) jeweils im Bereich einer jeweiligen Elektrode (3, 7) eine durch seine Dicke, von der Vorderseite zur Rückseite durchgehende Öffnung aufweist, durch welche die Elektrode (3, 7) leitend von der Vorderseite zur Rückseite des Trägers (2, 8) elektrisch kontaktiert ist.

2. Anordnung (1) nach Anspruch 1, **dadurch gekennzeichnet, dass** die Membrane (4) als eine geschlossene Schicht ausbildet ist, über welche die Elektroden (3, 7), insbesondere alle Elektroden (3, 7) miteinander verbunden sind.

3. Anordnung (1) nach einem der vorhergehenden Ansprüche, **dadurch gekennzeichnet, dass** die Membrane (4) ein Lateral Flow Papier, insbesondere aus Nitrocellulose umfasst.

4. Anordnung (1) nach einem der vorhergehenden Ansprüche, **dadurch gekennzeichnet, dass** die Elektroden (3, 7) Metallelektroden, insbesondere aus Gold sind.

5. Anordnung (1) nach einem der vorhergehenden Ansprüche, **dadurch gekennzeichnet, dass** der Träger (2, 8) mehrere isolierende Schichten umfasst, insbesondere Schichten aus Polymeren und/oder Schichten, welche miteinander durch laminieren verbunden sind.

6. Anordnung (1) nach einem der vorhergehenden Ansprüche, **dadurch gekennzeichnet, dass** die Membrane (4) sandwichartig angeordnet ist zwischen der Vorderseite des ersten Trägers (2) in direktem Kontakt mit den elektrisch leitenden Elektroden (3) des ersten Trägers (2) und einer Rückseite eines zweiten Trägers (8) in direktem Kontakt mit wenigstens einer Elektrode (7), insbesondere genau einer Elektrode (7) auf der Rückseite des zweiten Trägers (8).

7. Anordnung (1) nach Anspruch 6, **dadurch gekennzeichnet, dass** jede Elektrode (3) auf der Vorderseite des ersten Trägers (2) jeweils mit der wenigstens einen Elektrode (7) auf der Rückseite des zweiten Trägers (8) elektrisch verbunden ist, insbesondere über eine elektrische Messeinrichtung (10) zur Messung von Strom und/oder Spannung und/oder Kapazität.

8. Anordnung (1) nach einem der vorhergehenden Ansprüche, **dadurch gekennzeichnet, dass** die Elektroden (3) auf der Vorderseite des ersten Trägers (2) in einer Reihe (7) hintereinander oder in Array-Form angeordnet sind.

9. Verfahren zur elektrischen Detektion von flüssigen Proben (5) mit einer Anordnung (1) nach einem der vorhergehenden Ansprüche, wobei die flüssige Probe (5) auf die Membran aufgebracht wird und über insbesondere Kapillarkräfte über die Membran zu den Elektroden (3, 7) bewegt wird, und wobei die Membrane (4), insbesondere genau eine Membrane (4) die Elektroden (3, 7), insbesondere alle Elektroden (3, 7) untereinander verbindet,
**dadurch gekennzeichnet, dass**
der Träger (2, 8) jeweils im Bereich einer jeweiligen Elektrode (3, 7) eine durch seine Dicke, von der Vorderseite zur Rückseite durchgehende Öffnung aufweist, durch welche die Elektrode (3, 7) leitend von der Vorderseite zur Rückseite des Trägers (2, 8) elektrisch kontaktiert wird.

10. Verfahren nach Anspruch 9, **dadurch gekennzeichnet, dass** die Anordnung (1) nach dem Lateral Flow Verfahren eine räumliche und/oder zeitliche Auftrennung von Substanzen in der flüssigen Probe (5) bewirkt, welche mit den Elektroden (3, 7) in Form von Strom- und/oder Spannungs- und/oder Ladungsänderungen gemessen wird.

11. Verfahren nach einem der Ansprüche 9 oder 10, **dadurch gekennzeichnet, dass** die Membrane (4) in direktem Kontakt mit den Elektroden (3, 7) steht und jeweils die Kontaktfläche einer jeden Elektrode (3, 7) mit der Membrane (4) vollständig mit der flüssigen Probe (5) benetzt wird, insbesondere ohne Lufteinschlüsse.

12. Verfahren nach einem der Ansprüche 9 bis 11, **dadurch gekennzeichnet, dass** die flüssige Probe (5) eine biochemische Probe (5), insbesondere eine Körperflüssigkeit ist.

## Claims

1. Arrangement (1) for the electrical detection of liquid samples (5) by means of lateral flow assays, wherein the lateral flow assay comprises a membrane (4) arranged on a front side of a first carrier (2), and wherein the first carrier (2) is embodied in an electrically insulating fashion and electrically conductive electrodes (3) are arranged on the first carrier (2), and wherein the electrodes (3) on the front side of the first carrier (2) are arranged between the carrier (2) and the membrane (4), in direct contact with the membrane (4), **characterized in that**
the carrier (2, 8) has in each case in the region of a respective electrode (3, 7) an opening passing through its thickness, from the front side to the rear side, through which opening the electrode (3, 7) is electrically contact-connected conductively from the front side to the rear side of the carrier (2, 8).

2. Arrangement (1) according to Claim 1, **characterized in that** the membrane (4) is embodied as a closed layer via which the electrodes (3, 7), in particular all the electrodes (3, 7) are connected to one another.

3. Arrangement (1) according to either of the preceding claims, **characterized in that** the membrane (4) comprises a lateral flow paper, in particular composed of nitrocellulose.

4. Arrangement (1) according to any of the preceding claims, **characterized in that** the electrodes (3, 7) are metal electrodes, in particular composed of gold.

5. Arrangement (1) according to any of the preceding claims, **characterized in that** the carrier (2, 8) comprises a plurality of insulating layers, in particular layers composed of polymers and/or layers which are connected to one another by lamination.

6. Arrangement (1) according to any of the preceding claims, **characterized in that** the membrane (4) is arranged in a sandwich-like fashion between the front side of the first carrier (2) in direct contact with the electrically conductive electrodes (3) of the first carrier (2) and a rear side of a second carrier (8) in direct contact with at least one electrode (7), in particular exactly one electrode (7) on the rear side of the second carrier (8).

7. Arrangement (1) according to Claim 6, **characterized in that** each electrode (3) on the front side of the first carrier (2) is in each case electrically connected to the at least one electrode (7) on the rear side of the second carrier (8), in particular via an electrical measuring device (10) for measuring current and/or voltage and/or capacitance.

8. Arrangement (1) according to any of the preceding claims, **characterized in that** the electrodes (3) are arranged on the front side of the first carrier (2) in a series (7) in tandem or in array form.

9. Method for the electrical detection of liquid samples (5) by means of an arrangement (1) according to any of the preceding claims, wherein the liquid sample (5) is applied to the membrane and is moved by means of capillary forces, in particular, over the membrane to the electrodes (3, 7), and wherein the membrane (4), in particular exactly one membrane (4),interconnects the electrodes (3, 7), in particular all the electrodes (3, 7)
**characterized in that**
the carrier (2, 8) has in each case in the region of a respective electrode (3, 7) an opening passing through its thickness, from the front side to the rear side, through which opening the electrode (3, 7) is electrically contact-connected conductively from the front side to the rear side of the carrier (2, 8).

10. Method according to Claim 9, **characterized in that** the arrangement (1) brings about, according to the lateral flow method, a spatial and/or temporal separation of substances in the liquid sample (5) which is measured by means of the electrodes (3, 7) in the form of current and/or voltage and/or charge changes.

11. Method according to either of Claims 9 and 10, **characterized in that** the membrane (4) is in direct contact with the electrodes (3, 7) and in each case the contact area of each electrode (3, 7) with the membrane (4) is completely wetted with the liquid sample (5), in particular without air inclusions.

12. Method according to any of Claims 9 to 11, **characterized in that** the liquid sample (5) is a biochemical sample (5), in particular a body fluid.

## Revendications

1. Agencement (1) pour la détection électrique d'échantillons liquides (5) par des dispositifs de dosage à écoulement latéral, dans lequel le dispositif de dosage à écoulement latéral comporte une membrane (4) qui est agencée sur une face avant d'un premier support (2), et dans lequel le premier support (2) est conçu sous forme d'isolant électrique et des électrodes électriquement conductrices (3) sont agencées sur le premier support (2), et dans lequel les électrodes (3) sont agencées sur la face avant du premier support (2) entre le support (2) et la membrane (4), en contact direct avec la membrane (4), **caractérisé en ce que**
le support (2, 8), respectivement dans la zone d'une électrode respective (3, 7), comprend une ouverture continue selon son épaisseur, de la face avant à la face arrière, à travers laquelle l'électrode (3, 7) est mise en contact électrique de conduction de la face avant à la face arrière du support (2, 8).

2. Agencement (1) selon la revendication 1, **caractérisé en ce que** la membrane (4) est conçue sous la forme d'une couche fermée, par l'intermédiaire de laquelle les électrodes (3, 7), en particulier toutes les électrodes (3, 7), sont reliées les unes aux autres.

3. Agencement (1) selon l'une des revendications précédentes, **caractérisé en ce que** la membrane (4) comporte un papier à écoulement latéral, en particulier en nitrocellulose.

4. Agencement (1) selon l'une des revendications précédentes, **caractérisé en ce que** les électrodes (3, 7) sont des électrodes métalliques, en particulier en or.

5. Agencement (1) selon l'une des revendications précédentes, **caractérisé en ce que** le support (2, 8) comporte plusieurs couches isolantes, en particulier des couches en polymères et/ou des couches qui sont reliées les unes aux autres par stratification.

6. Agencement (1) selon l'une des revendications précédentes, **caractérisé en ce que** la membrane (4) est agencée en sandwich entre la face avant du premier support (2) en contact direct avec les électrodes électriquement conductrices (3) du premier support (2) et une face arrière d'un deuxième support (8) en contact direct avec au moins une électrode (7), en particulier exactement une électrode (7) sur la face arrière du deuxième support (8).

7. Agencement (1) selon la revendication 6, **caractérisé en ce que** chaque électrode (3) sur la face avant du premier support (2) est respectivement reliée électriquement à l'au moins une électrode (7) sur la face arrière du deuxième support (8), en particulier par l'intermédiaire d'un dispositif de mesure électrique (10) pour la mesure d'un courant et/ou d'une tension et/ou d'une capacité.

8. Agencement (1) selon l'une des revendications précédentes, **caractérisé en ce que** les électrodes (3) sur la face avant du premier support (2) sont agencées les unes derrière les autres en une rangée (7) ou sous la forme d'un réseau.

9. Procédé pour la détection électrique d'échantillons liquides (5) par un agencement (1) selon l'une des revendications précédentes, dans lequel l'échantillon liquide (5) est amené sur la membrane et est déplacé en particulier par des forces de capillarité à travers la membrane, jusqu'aux électrodes (3, 7), et dans lequel la membrane (4), en particulier exactement une membrane (4), relie les électrodes (3, 7), en particulier toutes les électrodes (3, 7) les unes aux autres,
**caractérisé en ce que**
le support (2, 8), respectivement dans la zone d'une électrode respective (3, 7), comprend une ouverture continue selon son épaisseur, de la face avant à la face arrière, à travers laquelle l'électrode (3, 7) est mise en contact électrique de conduction de la face avant à la face arrière du support (2, 8).

10. Procédé selon la revendication 9, **caractérisé en ce que** l'agencement (1) effectue selon le procédé à écoulement latéral une séparation spatiale et/ou temporelle de substances dans l'échantillon liquide (5), qui est mesurée par les électrodes (3, 7) sous la forme de modifications de courant et/ou de tension et/ou de charge.

11. Procédé selon l'une des revendications 9 ou 10, **caractérisé en ce que** la membrane (4) est en contact direct avec les électrodes (3, 7) et respectivement la surface de contact d'une électrode respective (3, 7) avec la membrane (4) est entièrement imprégnée de l'échantillon liquide (5), en particulier sans inclusions d'air.

12. Procédé selon l'une des revendications 9 à 11, **caractérisé en ce que** l'échantillon liquide (5) est un échantillon biochimique (5), en particulier un liquide corporel.
